(19) Europäisches Patentamt
European Patent Office
Office européen des brevets

(11) Veröffentlichungsnummer: **0 040 724**
**B1**

(12) # EUROPÄISCHE PATENTSCHRIFT

(45) Veröffentlichungstag der Patentschrift: **25.05.83**

(51) Int. Cl.³: **C 07 C 69/14,** C 07 C 67/03

(21) Anmeldenummer: **81103427.1**

(22) Anmeldetag: **06.05.81**

(54) **Verfahren zur kontinuierlichen Herstellung von Essigsäureestern.**

(30) Priorität: **23.05.80 DE 3019767**

(43) Veröffentlichungstag der Anmeldung:
**02.12.81 Patentblatt 81/48**

(45) Bekanntmachung des Hinweises auf die Patenterteilung:
**25.05.83 Patentblatt 83/21**

(84) Benannte Vertragsstaaten:
**BE DE FR GB IT NL**

(56) Entgegenhaltungen:
**DE-A-2 820 521**
**GE-A-1 113 970**
**GB-A-1 130 387**

(73) Patentinhaber: **BASF Aktiengesellschaft,**
**Carl-Bosch-Strasse 38, D-6700 Ludwigshafen (DE)**

(72) Erfinder: **Bott, Kaspar, Dr., Rieslingweg 4,**
**D-6706 Wachenheim (DE)**
Erfinder: **Kaibel, Gerd, Robert-Bosch-Strasse 4,**
**D-6840 Lampertheim (DE)**
Erfinder: **Hoffmann, Herwig, Dr., Knietschstrasse 21,**
**D-6710 Frankenthal (DE)**
Erfinder: **Irnich, Rudolf, Dr., In den Hahndornen 2,**
**D-6719 Bobenheim (DE)**
Erfinder: **Schaefer, Eberhard, Berliner Strasse 21C,**
**D-6700 Ludwigshafen (DE)**

**EP 0 040 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur kontinuierlichen Herstellung von Essigsäureestern

Die vorliegende Erfindung betrifft ein verbessertes Verfahren zur Herstellung von Essigsäureestern der allgemeinen Formel I

$$CH_3-CO-O-R^1 \qquad \text{I}$$

in welcher $R^1$ ein organischer Rest außer der Methyl- und der Ethylgruppe ist, durch alkali-katalysierte Umesterung eines Essigsäureesters der allgemeinen Formel II

$$CH_3-CO-O-R^2 \qquad \text{II}$$

in der $R^2$ die Methyl- oder Ethylgruppe bedeutet, mit einem Alkohol III

$$R^1-OH \qquad \text{III}$$

unter Abspaltung des Alkohols IV

$$R^2-OH \qquad \text{IV}$$

gemäß folgendem Reaktionsschema:

$$\underset{\text{II}}{CH_3-CO-O-R^2} + \underset{\text{III}}{R^1-OH} \rightleftarrows \underset{\text{I}}{CH_3-CO-O-R^1} + \underset{\text{IV}}{R^2-OH}$$

Dieses Umesterungsverfahren ist, sieht man von der erfindungsgemäßen Verbesserung ab, allgemein bekannt. Es wurde bisher im Prinzip so vorgenommen, daß man die Reaktion bis etwa zur Gleichgewichtseinstellung ablaufen ließ, wonach man das Reaktionsgemisch destillativ in seine Komponenten zerlegte. Äußerst nachteilig ist hierbei jedoch, daß Methanol und Methylacetat sowie Ethanol und Ethylacetat und meistens auch der Alkohol $R^1-OH$ und sein Acetat Azeotrope bilden. Aus diesem Grunde bereitet die destillative Aufarbeitung der Umesterungsgemische erhebliche Schwierigkeiten, und außerdem ist es auf wirtschaftliche Weise nicht möglich, das Veresterungsgleichgewicht durch laufende Entfernung des Alkohols $R^2-OH$ auf die Seite des gewünschten Esters I zu verschieben, da der Reaktion hierbei stets auch der Ausgangsester II (als Azeotrop mit IV) entzogen wird.

Aus der DE-A-2 820 521 war es speziell bekannt, höher siedende Alkohole, insbesondere Diole aus ihren Estern mit niederen Carbonsäuren wie Essigsäure durch Umesterung mit niederen Alkoholen wie Methanol und Entfernung des entstehenden niederen Esters herzustellen, indem man die Ausgangsstoffe in Gegenwart eines Umesterungskatalysators im Gleichstrom von oben durch eine Füllkörperkolonne führt, wobei man als Kopfprodukt den niederen Ester und als Sumpfprodukt den höheren Alkohol erhält. Dieses Prinzip, bei welchem die gewünschten Verfahrensprodukte die Reaktionspartner mit dem höchsten und tiefsten Siedepunkt sind, die sich beide mühelos zur gewünschten Gleichgewichtsverschiebung aus dem Gleichgewicht entfernen lassen, läßt sich jedoch nicht auf die Gewinnung der höheren Ester übertragen, eben weil nicht sie, sondern der höhere Alkohol als Sumpfprodukt anfallen.

Der Erfindung lag daher die Aufgabe zugrunde, diese Nachteile in einem kontinuierlichen Verfahren soweit zu beheben, daß das Azeotrop aus I und III nicht mehr störend in Erscheinung tritt, daß die Bildung der Azeotrope aus II und IV ebenfalls gänzlich unterdrückt wird oder, soweit dies nicht möglich ist, daß diese Azeotrope harmonisch in das Umesterungsverfahren einbezogen werden können.

Demgemäß wurde ein verbessertes Verfahren zur Herstellung der Essigsäureester I nach dem eingangs dargelegten Reaktionsprinzip gefunden, welches dadurch gekennzeichnet ist, daß man

a) die Umesterungsreaktion im Mittelteil ($K_M$) einer Destillationskolonne (K) vornimmt, wobei man den Alkohol III flüssig in den oberen Bereich und den Ester II in den unteren Bereich von ($K_M$) leitet,
und wobei man durch Einstellung entsprechend hoher Verweilzeiten und/oder durch Verwendung eines Überschusses des Esters II über den Alkohol III dafür sorgt, daß sich der Alkohol III vollständig umsetzt,
b) den alkalischen Katalysator in den Oberteil ($K_O$) von (K) einführt,
c) vom Kopf der Kolonne den Alkohol IV oder eine Mischung aus IV und dem Ester II entnimmt,
d) die nach (c) erhaltene Mischung (also sofern der Alkohol IV nicht allein anfällt) in dem Kolonnenteil ($K_O$)
oder in einer Nebenkolonne ($K_N$) in IV und das Azeotrop aus II und IV auftrennt und letzteres wieder in den unteren Bereich von ($K_M$) zurückführt,

e) den Ester I aus dem unteren Bereich des Unterteils ($K_U$) von (K) dampfförmig oder flüssig entnimmt und
f) den Katalysator auf übliche Weise entweder entfernt oder nach ($K_O$) zurückführt.

Das Verfahren sei anhand der Figur näher erläutert. Die Umesterungsreaktion findet im wesentlichen im Mittelteil ($K_M$) der Kolonne (K) statt, und zwar im Gegenstrom des Alkohols III und des Esters II. Hierbei entsteht der gewünschte Ester I, der als höher siedende Substanz in den Unterteil ($K_U$) der Kolonne gelangt. Damit man hier keine Mischung aus I und III erhält, muß man dafür sorgen, daß sich der Alkohol III vollständig umsetzt. Dies erreicht man dadurch, daß man in ($K_M$) entsprechend hohe Verweilzeiten einstellt und/oder daß man den Ester II im stöchiometrischen Überschuß über den Alkohol III einsetzt. Dieser Überschuß beträgt jedoch im allgemeinen nicht mehr als 0,1 bis 2 mol pro Mol III.

Setzt man den Ester II in reiner Form und nicht im Überschuß über III ein, so setzt er sich meistens vollständig um, so daß man am Kolonnenkopf praktisch den reinen Alkohol IV oder eine Mischung mit nur wenig II erhält. Die Fraktionierung in ($K_O$) oder die weitere Destillation in ($K_N$) erübrigt sich dann, sei es daß man IV einer beliebigen Verwendung zuführt oder, wie es besonders vorteilhaft ist, in die Stufe (der hier nicht gezeigten) Synthese von II zurückführt, denn das Methylacetat wird man üblicherweise durch Carbonylierung von Methanol und das Ethylacetat nach Tischtschenko aus Ethanol herstellen. Bei beiden Synthesen stören geringe Mengen der Ester II naturgemäß nicht.

Setzt man II jedoch im Überschuß über III ein oder enthält II noch merkliche Anteile von IV, wie es für die Praxis zutrifft, erhält man am Kolonnenkopf eine Mischung aus IV und größeren Mengen II. Diese Mischung wird in der Kolonne ($K_N$) oder auch in dem Kolonnenteil ($K_O$) in den reinen Alkohol IV und das Azeotrop aus IV und II zerlegt. Der reine Alkohol IV kann dann wieder beliebig weiterverwendet oder in die Synthese von II zurückgeführt werden, und das Azeotrop wird wieder in den unteren Bereich von ($K_M$) zurückgeführt.

Als alkalische Katalysatoren verwendet man vorzugsweise Alkalialkoholate, insbesondere $Na-O-R^2$ oder $K-O-R^2$, in Form von 0,1- bis 0,5gew.-%igen Lösungen im Alkohol $R^2-OH$ (IV), wobei die Menge des Katalysators zweckmäßigerweise 0,05 bis 1 Mol-% des eingesetzten Alkohols III beträgt.

Da auch ein Teil des Alkohols III in den oberen Kolonnenteil ($K_O$) gelangt, kann hier ebenfalls noch die Umesterung in geringem Umfang stattfinden. Aus diesem Grunde empfiehlt es sich, den Katalysator im oberen Bereich vom ($K_O$) zuzuführen. Für Versuchszwecke genügt es jedoch meistens, den Katalysator zusammen mit dem Alkohol III in die Kolonne zu geben.

Entnimmt man den Ester I dampfförmig aus ($K_U$), so sammelt sich im Sumpf eine katalysatorhaltige Lösung an, die in den Kreislauf zurückgeführt werden kann. Da sich der Katalysator aber im Laufe der Umesterungsreaktion meistens verbraucht, indem sich das weniger wirksame Alkaliacetat bildet, ist es im allgemeinen vorteilhafter, das katalysatorhaltige Sumpfprodukt zu verwerfen.

Wenn man den Ester I flüssig entnimmt, wird der Katalysator zusammen mit dem Ester entfernt. In diesem Falle muß der Ester gereinigt werden, indem man ihn mit Wasser oder verdünnter Säure auswäscht. Selbstverständlich ist es im Falle der flüssigen Entnahme des Esters I auch möglich, diesen in einer Seitenkolonne destillativ vom Katalysator zu befreien.

Das erfindungsgemäße Umesterungsverfahren eignet sich prinzipiell zur Herstellung beliebiger Ester I, sofern diese und der dazugehörige Alkohol III unter den Reaktionsbedingungen flüssig sind. Die Reste $R^1$ in I bzw. III können daher aliphatische, cycloaliphatische, araliphatische und aromatische Reste mit bis zu 12 C-Atomen sein, die ihrerseits inerte Substituenten wie Halogen und Ester- oder Polyethergruppierungen enthalten können. Besonders wichtige Ester I sind solche, die als Lösungsmittel Verwendung finden. Diese Ester leiten sich von $C_2-C_{12}$-Alkanolen und von (Poly-)Glykolethern des Typs $R'-O-(A-O)_n-H$ ab, wobei R' eine $C_1-C_4$-Alkylgruppe bedeutet, A für die Ethylen- oder 1,2-Propylengruppe steht und n einen Wert von 1 bis 4 hat. Eine weitere Klasse wichtiger Ester I sind solche, die als Duft- und Aromastoffe Verwendung finden. Derartige Ester leiten sich z. B. vom Amylalkohol, Isoamylalkohol, Benzylalkohol und Terpenalkoholen wie Linalool und Citronellol ab.

Das Herzstück der Kolonne (K) ist deren Mittelteil ($K_m$), in welchem die Umesterungsreaktion vorwiegend stattfindet. Dieser Kolonnenteil, der im allgemeinen 8 bis 20 theoretische Trennstufen aufweisen soll, ist vorzugsweise als Glocken-, Ventil- oder Siebbodenkolonne ausgebildet, da diese Bauarten die Einstellung hoher Verweilzeiten, wie sie für die Umesterungsreaktion meist erforderlich sind, gestattet.

Vielfach reichen aber auch Füllkörperkolonnen für die Zwecke des Kolonnenteils ($K_m$) aus. Der obere Kolonnenteil ($K_O$) sowie der unteren Kolonnenteil ($K_U$) haben dagegen vorwiegend die Funktion nicht sonderlich schwieriger Stofftrennungen, weshalb hier einfache Bauarten wie Füllkörperkolonnen in aller Regel genügen. Es kann sich jedoch empfehlen, auch diese Kolonnenteile als Glocken-, Ventil- oder Siebbödenkolonnen auszulegen, weil man hierdurch die Abgrenzung der drei Kolonnenteile flexibel gestalten und den unterschiedlichsten Umesterungsaufgaben leicht anpassen kann. Im allgemeinen sollen ($K_O$) und ($K_U$) 3 bis 15 theoretische Böden aufweisen.

Es ist vorteilhaft, das Verfahrensprodukt, also den Ester I, als dampfförmigen Seitenabzug von ($K_U$)

abzuziehen, da der Ester auf diese Weise unmittelbar in reiner Form anfällt und da seine Kondensationswärme zur Verdampfung des eingesetzten Esters II ausgenutzt werden kann. Selbstverständlich kann man den Ester I mit gleichem Erfolg auch in einer Nebenkolonne destillieren oder fraktionieren.

Prinzipiell kann man das erfindungsgemäße Umesterungsverfahren bei beliebigem Druck ausführen, jedoch bevorzugt man das Arbeiten bei Normaldruck oder einem solchen leicht erhöhten Druck (etwa bis zu 3 bar), der die Kondensation der niedrig siedenden Ester II und Alkohole IV mittels normalem Kühlwasser gestattet.

## Beispiel

### Herstellung von n-Butylacetat aus Methylacetat

Für die Herstellung von n-Butylacetat durch Umesterung von Methylacetat mit n-Butanol bei Normaldruck diente eine Versuchskolonne mit etwa 50 theoretischen Trennstufen. Diese Kolonne war 3 m hoch, hatte einen Innendurchmesser von 5 cm und war mit Maschendrahtringen von 0,5 cm Durchmesser gefüllt.

Auf Höhe des 15. Bodens (von unten gezählt) wurde bei 91°C stündlich ein azeotropes Gemisch aus 328 g Methylacetat und 82 g Methanol dampfförmig eingeleitet, und auf Höhe des 35. Bodens (84°C) wurde der Kolonne stündlich eine Lösung aus 112 g n-Butanol und 0,5 g K-butylat zugeführt. Der Mittelteil ($K_M$) der Kolonne war somit durch die Böden 15 und 35 definiert.

Am Kolonnenkopf (54°C) fiel bei einem Rücklaufverhältnis von 5 stündlich ein Gemisch aus 129 g Methanol und 219 g Methylacetat an, welches in einer Seitenkolonne (35 theoretische Böden) in das dampfförmige Azeotrop aus 55 g Methanol und 219 g Methylacetat einerseits und in reines flüssiges Methanol (74 g) andererseits zerlegt wurde. Das Azeotrop wurde dampfförmig auf den Boden 15 der Kolonne zurückgeführt.

Am unteren Kolonnenende (129°C) wurden stündlich 169 g n-Butylacetat, welches noch etwa 2 Gew.-% n-Butanol enthielt, dampfförmig abgezogen. Den Sumpf bildeten 5 g n-Butylacetat pro Stunde, welche den Katalysator enthielten. Dieses Sumpfprodukt wurde keiner weiteren Aufarbeitung unterworfen.

Hieraus ergibt sich, daß das n-Butylacetat in 98%iger Reinheit in einer Ausbeute von 97% anfiel.

## Patentanspruch

Verfahren zur Herstellung von Essigsäureestern der allgemeinen Formel I

$$CH_3-CO-O-R^1 \qquad I$$

in welcher $R^1$ ein organischer Rest außer der Methyl- und der Ethylgruppe ist, durch alkali-katalysierte Umesterung eines Essigsäureesters der allgemeinen Formel II

$$CH_3-CO-O-R^2 \qquad II$$

in der $R^2$ die Methyl- oder Ethylgruppe bedeutet, mit einem Alkohol III

$$R^1-OH \qquad III$$

unter Abspaltung des Alkohols IV

$$R^2-OH \qquad IV$$

dadurch gekennzeichnet, daß man

a) die Umesterungsreaktion im Mittelteil ($K_M$) einer Destillationskolonne (K) vornimmt, wobei man den Alkohol III flüssig in den oberen Bereich und den Ester II in den unteren Bereich von ($K_M$) leitet,
und wobei man durch Einstellung entsprechend hoher Verweilzeiten und/oder durch Verwendung eines Überschusses des Esters II über den Alkohol III dafür sorgt, daß sich der Alkohol III vollständig umsetzt,
b) den alkalischen Katalysator in den Oberteil ($K_O$) von (K) einführt,
c) vom Kopf der Kolonne den Alkohol IV oder eine Mischung aus IV und dem Ester II entnimmt,
d) die nach (c) erhaltene Mischung (also sofern der Alkohol IV nicht allein anfällt) in dem Kolonnenteil ($K_O$) oder in einer Nebenkolonne ($K_N$) in IV und das Azeotrop aus II und IV auftrennt

und letzteres wieder in den unteren Bereich von ($K_M$) zurückführt,

e) den Ester I aus dem unteren Bereich des Unterteils ($K_U$) von (K) dampfförmig oder flüssig entnimmt und

f) den Katalysator auf übliche Weise entweder entfernt oder nach ($K_O$) zurückführt.

**Claim**

A process for the preparation of an acetic acid ester of the general formula I

$$CH_3-CO-O-R^1 \qquad I$$

where $R^1$ is an organic radical other than methyl or ethyl, by alkali-catalyzed trans-esterification of an acetic acid ester of the general formula II

$$CH_3-CO-O-R^2 \qquad II$$

where $R^2$ is methyl or ethyl, with an alcohol III

$$R^1-OH \qquad III$$

accompanied by elimination of the alcohol IV

$$R^2-OH \qquad IV$$

wherein

a) the trans-esterification reaction is carried out in the middle section ($K_M$) of a distillation column (K), the alcohol III being fed as liquid into the upper zone and the ester II into the lower zone ($K_M$), and the selection of an appropriately long residence time and/or the use of an excess of the ester II over the alcohol III ensuring that the alcohol III reacts completely,

b) the alkaline catalyst is introduced into the upper part ($K_U$) of (K),

c) the alcohol IV, or a mixture of IV and the ester II, is taken off the top of the column,

d) the mixture obtained from (c) (unless the alcohol IV alone is obtained) is separated in the column section ($K_U$) or in a stripper column ($K_S$) into IV and the azeotrope of II and IV, and the latter is recycled to the lower zone of ($K_M$),

e) the ester I is taken as vapor or liquid from the lower zone of the lower section ($K_L$) of (K), and

f) the catalyst is either removed, or recycled to ($K_U$), in a conventional manner.

**Revendication**

Procédé de préparation d'esters de l'acide acétique de la formule générale I

$$CH_3-CO-O-R^1 \qquad (I)$$

dans laquelle $R^1$ désigne un groupe organique autre qu'un radical méthyle ou éthyle, par une transestérification, catalysée par un alcali, d'un ester de l'acide acétique de la formule générale II

$$CH_3-CO-O-R^2 \qquad (II)$$

dans laquelle $R^2$ désigne un radical méthyle ou éthyle, à l'aide d'un alcool III

$$R^1-OH \qquad (III)$$

avec séparation d'un alcool IV

$$R^2-OH \qquad (IV)$$

caractérisé en ce que:

a) on procède à la réaction de transestérification dans la région médiane ($K_M$) d'une colonne de distillation (K), l'alcool III étant introduit à l'état liquide dans la partie supérieure et l'ester II dans la partie inférieure de la région ($K_M$),
la transformation complète de l'alcool III étant obtenue, soit par des durées de séjour

suffisamment longues, soit par la mise en oeuvre de l'ester II en excès par rapport à l'alcool III;

b) on introduit le catalyseur alcalin dans la région supérieure ($K_O$) de la colonne (K);

c) on soutire en tête de la colonne l'alcool IV ou un mélange d'alcool IV et d'ester II;

d) lorsque le produit obtenu en c) est un mélange, on sépare celui-ci dans la région ($K_O$) de la colonne (K), ou dans une colonne secondaire ($K_N$), en alcool IV et en azéotrope de II et de IV, qui est recyclé dans la partie inférieure de la région médiane ($K_M$);

e) on soutire l'ester I à l'état liquide ou gazeux de la partie inférieure de la région inférieure ($K_U$) de la colonne (K) et

f) on élimine le catalyseur de manière usuelle ou on le recycle dans la région ($K_O$).

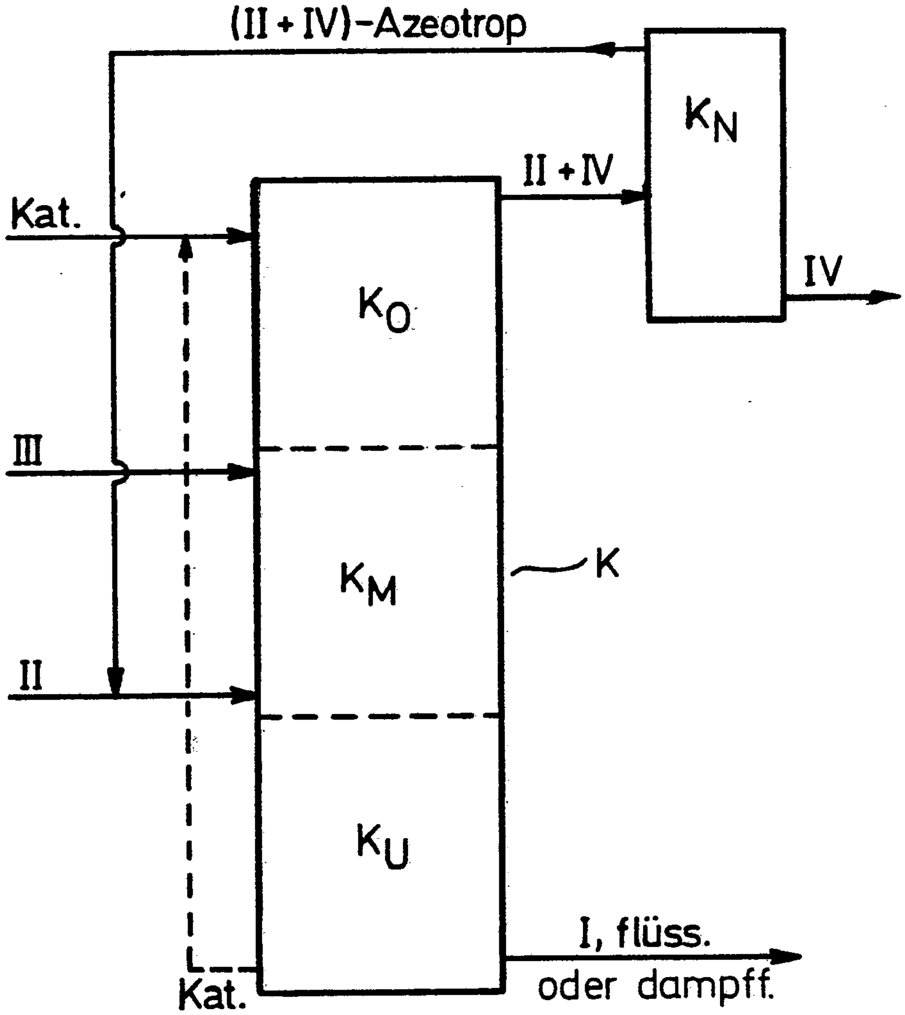


$$\underset{II}{CH_3-CO-O-R^2} + \underset{III}{R^1-OH} \xrightarrow{Kat.} \underset{I}{CH_3-CO-O-R^1} + \underset{IV}{R^2-OH}$$